(19) Europäisches Patentamt / European Patent Office / Office européen des brevets

(11) **EP 4 564 364 A1**

(12) **EUROPEAN PATENT APPLICATION**

(43) Date of publication:
04.06.2025  Bulletin 2025/23

(21) Application number: 24203402.3

(22) Date of filing: 27.09.2024

(51) International Patent Classification (IPC):
*G16H 30/40* (2018.01)     *G16H 50/20* (2018.01)

(52) Cooperative Patent Classification (CPC):
G16H 30/40; G16H 50/20

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.11.2023  KR 20230168898
27.05.2024  KR 20240068620

(71) Applicant: Lunit Inc.
Seoul 06241 (KR)

(72) Inventors:
• NAM, Gunhee
06241 Seoul (KR)
• JOO, Won Hyeong
06241 Seoul (KR)

(74) Representative: Germain Maureau
12, rue Boileau
69006 Lyon (FR)

(54) **METHOD AND SYSTEM FOR ARTIFICIAL INTELLIGENCE-BASED MEDICAL IMAGE ANALYSIS**

(57) An image analysis device includes a memory and a processor configured to execute instructions stored in the memory, wherein the processor is configured to detect an indeterminate region or an abnormal region from an input medical image using an artificial intelligence (AI) model trained to detect a suspicious region and a lesion region in medical images and determine the input medical image as a normal case when the indeterminate region or the abnormal region is not detected in the input medical image.

EP 4 564 364 A1

**Description**

## CROSS-REFERENCE TO RELATED APPLICATION

This application claims priority to and the benefit of Korean Patent Application No. 10-2023-0168898 filed in the Korean Intellectual Property Office on November 29, 2023, and Korean Patent Application No. 10-2024-0068620 filed in the Korean Intellectual Property Office on May 27, 2024, the entire contents of which are incorporated herein by reference.

## BACKGROUND

### (a) Field of the Invention

The disclosure relates to an artificial intelligence (AI)-based medical image analysis technology.

### (b) Description of the Related Art

Recently, as artificial intelligence (AI) technology has been increasingly adopted in the medical field, leading to AI-based medical image analysis technology, such as the Lunit INSIGHT solution. These solutions enable AI to analyze medical images and provides the analysis results in a visual format.

A radiologist may receive an image reading task through a worklist, review abnormalities provided by medical image analysis, and then proceed with generating a radiologist report. The radiologist may prioritize images that require urgent reading or images where abnormalities have been detected. However, this approach only adjusts the reading order of images in the worklist. As a result, the reading of normal images must still be performed eventually, meaning the overall workload for the radiologist remains unchanged.

## SUMMARY

The disclosure attempts to provide an artificial intelligence (AI)-based medical image analysis method and system.

The disclosure relates to an interface screen that provides AI-based medical image analysis results.

The disclosure relates to a method for providing a worklist using AI-based medical image analysis results.

The disclosure relates to a method for automatically generating a report using AI-based medical image analysis results.

According to an exemplary embodiment, an image analysis device includes a memory and a processor configured to execute instructions stored in the memory. The processor is configured to detect an indeterminate region or an abnormal region from an input medical image using an artificial intelligence (AI) model trained to detect a suspicious region and a lesion region in medical images, and determine the input medical image as a normal case when the indeterminate region or the abnormal region is not detected in the input medical image.

The AI model may be trained to detect the suspicious region using suspicious images and non-suspicious images and to detect the lesion region using lesion images including a target lesion and non-lesion images not including the target lesion. The suspicious images may include the lesion images and images including a lesion suspected as the target lesion.

The processor may be configured to obtain a suspiciousness score related to a confidence level at which the suspicious region is certain and a lesion score related to a confidence level at which the lesion region is certain using the AI model, and classify regions of the input medical image into one of normal, indeterminate, or abnormal based on analysis results of the input medical image including the suspiciousness score and the lesion score.

The processor may be configured to determine the input medical image as the normal case when the entire regions of the input medical image are classified as being normal.

The processor may be configured to calculate a rearrangement score based on the suspiciousness score and the lesion score, and obtain a classification result corresponding to the rearrangement score by using a first threshold value that classifies each region as being normal or indeterminate and a second threshold value that classifies each region as being indeterminate or abnormal.

The processor may be configured to determine the highest rearrangement score analyzed from the input medical image as an abnormality score of the corresponding medical image.

The processor may be configured to provide analysis results and normal case information for the input medical image obtained by the AI model to a designated device.

The processor may be configured to provide the analysis results for the input medical image in at least one of forms of secondary capture (SC) of a DICOM format or grayscale softcopy presentation state (GSPS). The SC or the GSPS for the normal case may include a graphical indicator indicating that the input medical image is the normal case.

The processor may be configured to provide the analysis results for the input medical image in at least one of the forms of SC of the DICOM format or GSPS. The SC or the GSPS may distinguishably display the indeterminate region and the abnormal region.

According to another exemplary embodiment, an method of an image analysis device includes classifying regions of an input medical image into one of normal, indeterminate, or abnormal region using an artificial intelligence (AI) model trained to detect a suspicious region and a lesion region in a medical image, and determining the input medical image as a normal case when the indeterminate region or the abnormal region is not detected in the input medical image.

The AI model may be trained to detect the suspicious region using suspicious images and non-suspicious images and to detect the lesion region using lesion images including a target lesion and non-lesion images not including the target lesion. The suspicious images may include the lesion images and images including a lesion suspected of being the target lesion.

The classifying regions of the input medical image may include obtaining a suspiciousness score related to a confidence level at which the suspicious region is certain and a lesion score related to a confidence level at which the lesion region is certain, using the AI model, and classifying regions of the input medical image into one of normal, indeterminate, or abnormal based on analysis results of the input medical image including the suspiciousness score and the lesion score. The classifying regions of the input medical image may include calculating a rearrangement score based on the suspiciousness score and the lesion score, and obtaining a classification result corresponding to the rearrangement score by using a first threshold value that classifies each region as being normal or indeterminate and a second threshold value that classifies each region being indeterminate or abnormal.

The method may further include: determining a highest rearrangement score analyzed from the input medical image as an abnormality score of the corresponding medical image.

The method may further include providing analysis results and normal case information for the input medical image obtained by the AI model to a designated device.

The analysis results for the input medical image may be provided in at least one of forms of secondary capture (SC) of a DICOM format or grayscale softcopy presentation state (GSPS). The SC or the GSPS for the normal case may include a graphical indicator indicating that the input medical image is the normal case.

The analysis results for the input medical image may be provided in at least one of the forms of SC of the DICOM format or GSPS. The SC or the GSPS may distinguishably display the indeterminate region and the abnormal region.

According to another exemplary embodiment, a computer program stored in a computer-readable recording medium includes instructions causing a processor to display a worklist including a study case list for image reading task by interworking with an image storage device storing analysis results for medical images, and when a specific medical image is determined to be a normal case based on normal case information included in the analysis results for the medical images, display an indicator indicating that the specific medical image is a normal case in the image list or in a preview image of the specific medical image, or display an indicator indicating that there is a pre-generated report for the specific medical image.

The computer program may further include instructions causing the processor to, in response to a selection of a first medical image, which is the normal case, from the worklist, display a graphical indicator indicating the normal case on a secondary capture (SC) image or grayscale softcopy presentation state (GSPS) representing an analysis result of the first medical image, and in response to a selection of a second medical image, which is an abnormal case, from the worklist, display an indeterminate region and an abnormal region distinguishably on the SC image or GSPS representing an analysis result of the second medical image.

The computer program may further include instructions causing the processor to
display the pre-generated report for the specific medical image in response to a user input; and store a report approved by the user.

According to an exemplary embodiment, the accuracy and reliability of analysis results may be improved by analyzing medical images using an AI model trained to analyze medical images with different tasks.

According to an exemplary embodiment, by classifying normal cases with high reliability among medical images and providing a graphical indicator that may recognize normal cases, the reading time and reading workload may be reduced, thereby improving reading efficiency.

According to an exemplary embodiment, by classifying normal cases with high reliability among medical images, a medical institution using the same may reduce reading costs.

According to an exemplary embodiment, an indeterminate region may be detected, and an indeterminate region requiring further attention may be displayed through this.

According to an exemplary embodiment, by displaying a suspicious region that is not definitively identified as a lesion, such as cancer, but has a lesion score lower than a threshold value as an indeterminate region, a user may be supported to check the indeterminate region without missing it, and as a result, the accuracy and reliability of the medical image analysis system may be improved.

According to an exemplary embodiment, by assigning the reading task for a normal case to less-skilled users or a remote reading company, the reading task within the hospital may be efficiently operated, thereby reducing memory resources and computing resources of the medical imaging system for managing medical images awaiting reading.

According to an exemplary embodiment, the user may identify a normal case in a worklist including a study case list for image reading task to prioritize abnormal cases for reading ahead of normal cases.

According to an exemplary embodiment, the user may allocate more time for interpreting an abnormal case than a normal case, reducing the time spent on normal cases by using a secondary capture (SC) image including an indication that it is a normal case, a grayscale softcopy presentation state (GSPS), etc.

According to an exemplary embodiment, by automatically generating a report based on analysis results of a medical image, the reading time and workload of the user (radiologist) may be reduced, thereby improving the reading efficiency, and as a result, memory resources and computing resources of the medical imaging system for managing medical images waiting for reading may be reduced.

According to an exemplary embodiment, by utilizing the analysis results of medical images not only in PACS but also in medical information systems, such as EMR, medical institution processes including diagnosis, treatment, and issuance of radiologist report may be remarkably improved, and as a result, a medical information system that processes the medical institution processes may be improved.

According to an exemplary embodiment, analysis results for mammography images may be obtained using an AI model, and reports, such as a radiologist report created by a medical imaging expert (radiologist) are automatically generated for normal cases using an automatic report creation function, so that even medical institutions without medical imaging experts may adopt mammography equipment and provide treatment.

## BRIEF DESCRIPTION OF THE DRAWINGS

FIG. 1 is a diagram of a medical imaging system according to an exemplary embodiment.

FIG. 2 is a diagram illustrating a medical image classification method according to an exemplary embodiment.

FIGS. 3 to 5 are examples of images providing analysis results of medical images according to an exemplary embodiment.

FIG. 6 is an example of a method for displaying lesion regions according to an exemplary embodiment.

FIG. 7 is an example of an interface for setting an analysis result display method according to an exemplary embodiment.

FIG. 8 is an example of a worklist according to an exemplary embodiment.

FIG. 9 is an example of an automatically generated report according to an exemplary embodiment.

FIG. 10 is an example of a report setting interface according to an exemplary embodiment.

FIG. 11 is an example of a worklist providing a preview image according to an exemplary embodiment.

FIG. 12 is an example of an image providing interface of a normal case according to an exemplary embodiment.

FIG. 13 is a flowchart of an image analysis method according to an exemplary embodiment.

FIG. 14 is a flowchart of a method of automatically generating a report according to an exemplary embodiment.

FIG. 15 is a flowchart of an analysis result providing method according to an exemplary embodiment.

## DETAILED DESCRIPTION OF THE EXEMPLARY EMBODIMENTS

Hereinafter, exemplary embodiments of the disclosure will be described in detail with reference to the accompanying drawings so that those of ordinary skill in the art may easily implement the exemplary embodiments. The exemplary embodiments may, however, be embodied in many different forms and is not limited to the exemplary embodiments set forth herein. In the drawings, parts irrelevant to the description are omitted to clearly describe the disclosure, and like reference numerals denote like elements throughout the specification.

When a certain part is referred to as "including" a certain component, this does not exclude other components unless described otherwise, and other components may be further included. In addition, the terms "-er", "-or", and "module" described in the specification mean units for processing at least one function and operation, and may be implemented by hardware components or software components, and combinations thereof.

In the description, a device or a terminal is a computing device configured and connected so that at least one processor may perform operations of the disclosure by executing instructions. The computer program includes instructions that are described for a processor to execute the operations of the disclosure, and may be stored in a non-transitory computer readable storage medium. The computer program may be downloaded via a network or sold as a product.

Medical images of the disclosure may be images of various body parts taken by various modalities, and for example, the modalities may be X-ray, magnetic resonance imaging (MRI), ultrasound, computed tomography (CT), digital mammography (MMG), digital breast tomosynthesis (DBT), etc.

A user of the disclosure may be a medical professional, such as a doctor, nurse, clinical pathologist, sonographer, or radiologist, but is not limited thereto.

The AI model (AI model) of the disclosure is a model that learns at least one task and may be executed by a processor. The task learned by the AI model may refer to a task to be solved through learning or a task to be performed through learning. The AI model may be implemented as a computer program that is executed on a computing device, may be downloaded through a network, or sold in the form of a product. Alternatively, the AI model may be linked with various devices through a network.

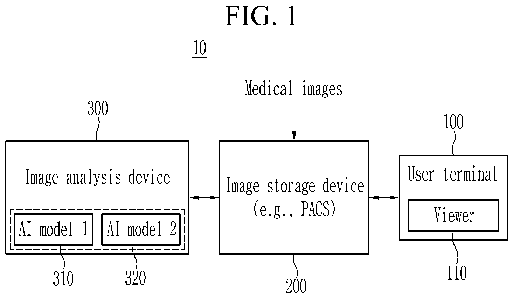

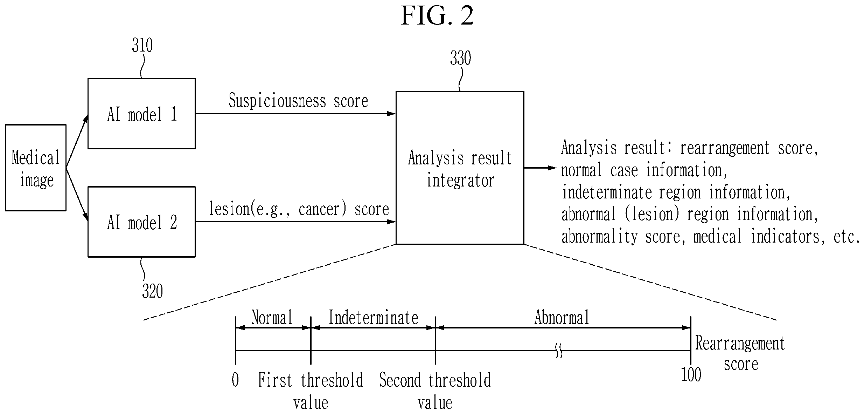

FIG. 1 is a diagram of a medical imaging system according to an exemplary embodiment, and FIG. 2 is a diagram illustrating a medical image classification method according to an exemplary embodiment.

Referring to FIG. 1, a medical imaging system 10 may include at least one user terminal 100, an image storage device 200, and an image analysis device 300. The interconnection between the devices constituting the medical imaging system 10 may be variously designed and changed. The medical imaging system 10 may be implemented to be interconnected with various medical information systems.

The user terminal 100 installs programs executed by a processor and includes hardware and software that provides a computing environment and a network environment for performing the operations of the disclosure.

The user terminal 100 may be implemented in various types, for example, a computing device in a workstation, a mobile device, etc. The user terminal 100 may include a viewer 110 that displays medical image-related data stored in the image storage device 200 by interworking with the image storage device 200 (simply referred to as a "viewer"). The viewer 110 may be installed and operated on, for example, a computing device in a workstation, may be implemented to access the image storage device 200, and may display medical image-related data stored in the image storage device 200. The viewer 110 is a computer program stored on a computer-readable medium and includes instructions executed by a processor. The processor in the user terminal 100 may perform operations described in the disclosure by executing the instructions.

The viewer 110 may display image analysis results stored in the image storage device 200. The viewer 110 may provide a worklist that lists a list of images that the user has to read along with key information. The viewer 110 may include a picture archiving and communication system (PACS) viewer. Here, the viewer 110 is a program that is designed to display medical images and/or image analysis results stored in the image storage device 200, and may support image reading task associated with the worklist, but is not limited to a viewer for reading task.

The image storage device 200 may store and manage taken medical images. Also, the image storage device 200 may store and manage analysis results for medical images. The image storage device 200 may include a PACS database. The image storage device 200 may store data according to a designated data format. For example, the image storage device 200 may store medical images taken by medical imaging devices (modalities) according to a digital imaging and communications in medicine (DICOM) standard and analysis results of medical images and may communicate with the user terminal 100 to provide data for image reading. The image storage device 200 and the viewer 110 may be configured as a PACS system, and the image storage device 200 may be a PACS server/DB and the viewer 110 may be a PACS viewer.

In this disclosure, the DICOM standard used for storing medical images is described as an example, but the medical image standard does not need to be limited to DICOM.

The image storage device 200 may obtain analysis results of medical images from the image analysis device 300. The analysis results of the medical images may include various medical predictions including lesion information. The analysis results of these medical images may be provided to assist the user in interpreting the images. The analysis results of the medical images may be provided in various formats, for example, secondary capture (SC) of DICOM format, grayscale softcopy presentation state (GSPS), etc. SC is to generate an image separate from the original medical image and display analysis results, may be provided separately from the original medical image, and may be displayed in the PACS viewer. GSPS may display lesion information on the original medical image in an overlay manner, may turn on/off the overlaid lesion information, and may be displayed in the PACS viewer. That is, when the image analysis device 300 transmits the analysis result of the medical image in a designated format (e.g., annotation and text), the image storage device 200 may provide GSPS by overlaying the analysis result on the original medical image. In the description, the SC image may be mainly used as an example of an image that provides the analysis result. In addition, the analysis result of the medical image may also be provided as a report in a text format. The report may include a radiologist report. For example, the report may be generated according to DICOM Basic Text Structured Report (SR), or HL7 Message. In addition, the analysis result of the medical image may be provided in various formats, and in particular, may be provided in various DICOM formats.

Medical images stored in the image storage device 200 may include images obtained by medical imaging devices of various modalities for various body parts. The medical images may be X-ray images, magnetic resonance imaging (MRI) images, ultrasound images, computed tomography (CT) images, digital mammography (MMG) images, digital breast tomosynthesis (DBT) images, etc. In the description, digital MMG images or DBT images are described as examples of medical images, but medical images need not be limited thereto, and the disclosure may be applied to a variety of medical images.

The image analysis device 300 may analyze medical images using an AI model and store the analysis results in the image storage device 200.

The image analysis device 300 may include an AI model specialized for each type of medical image and may perform analysis, such as lesion detection by selecting an AI model according to the type of input image. The AI model may be implemented to make medical inferences from medical images, and a model structure, training data configuration, training method, and medical inference target may be designed variously.

The image analysis device 300 may obtain analysis results of medical images using an AI model trained to analyze medical images according to multiple tasks and generate final analysis results for the medical images based on the analysis results. The image analysis device 300 may detect an indeterminate region or lesion region in an input medical image using an AI model trained to detect a suspicious region and a lesion region in the medical images, and if the indeterminate region and the lesion region is not detected in the input medical images, the image analysis device 300 may classify the input medical image as a normal case.

In order to describe separately that the AI modes analyze and output medical images according to different tasks, it is assumed that the image analysis device 300 uses AI model 1 310 and the AI model 2 320. However, the AI model used in the image analysis device 300 may not need to be clearly distinguished into AI model 1310 and the AI model 2 320 physically or logically, and the AI model implementing the disclosure may be designed in various forms. In addition, the number of AI models classified by task does not necessarily have to be limited to the two models.

A report may be automatically generated based on the analysis results of medical images. In the description, it is assumed that the image analysis device 300 automatically generates the report, and the report may be generated by a device (report generating device) that may access the analysis results or an AI model (report generating model) trained to generate the report. The automatically generated report may be stored in the image storage device 200, and the device in which the report is stored may be defined variously. In the following, it is described that a report is automatically generated for a normal case among medical images, and the image analysis device 300 may generate a report based on the analysis results of medical images in addition to the normal case, thereby reducing the time it takes for the user to create a report. A timing for automatically generating the report may be determined variously. For example, a report may be generated when a medical image is classified as a normal case, a report may be generated when the normal case is assigned to a specific user among multiple users, and a report may be generated when the user requests the normal case report from the worklist, but it is not necessary to be limited thereto.

If the medical image is a normal case, the user provides a patient with a radiologist report stating that it is normal. Since the normal case has no special findings, the same content is generally written. Therefore, the image analysis device 300 may pregenerate a report with phrases set by the user for the medical image analyzed as a normal case through a report automatic creation function. The phrases may be created and changed by the user, and the report may be automatically generated with phrases corresponding to the analysis results of the medical image. Meanwhile, even for a normal case, the report needs to be generated to be different depending on an analysis target and/or analysis result. For example, if the medical image is a mammography image, phrases according to breast density may be set in advance, and a report may be generated using phrases related to the analyzed breast density.

The image analysis device 300 may transmit the analysis results of the medical image to a designated device, for example, the image storage device 200, such as a PACS. The image analysis device 300 may generate a report for the medical image that is a normal case and transmit the report to the designated device.

The image analysis device 300 may transmit the analysis results to a medical information system, such as an electronic medical record (EMR) system or an electronic health record (EHR) system. For example, the image analysis device 300 may be implemented to interwork with a medical information system, such as an EMR through a standard (e.g., an HL7 message) established to enable information compatibility between medical information systems and may transmit analysis results to the medical information system. Through this, the medical information system may improve the medical work process by utilizing the AI analysis results. For example, if the patient's medical image is analyzed as a normal case, the user may access the EMR system, check an automatically generated radiologist report, and then approve it to complete the treatment, and the approved report may be recorded in the patient chart of the medical information system.

Referring to FIG. 2, the image analysis device 300 may analyze the medical image through an AI model trained to perform multiple tasks, and synthesize the analysis results to generate the final analysis results. The image analysis device 300 may detect a suspicious region in a medical image and may also use an AI model trained to detect a target lesion (e.g., cancer) in a medical image. The AI model that detects a target lesion (e.g., cancer) in the medical image may be further trained to detect a suspicious region. For the purpose of description, it is assumed that the image analysis device 300 analyzes a medical image through the AI model 1 310 trained to detect a suspicious region and the AI model 2 320 trained to detect a specific lesion, and an analysis result integrator (referred to simply as the integrator) 330 generates an integrated analysis result. In addition, the image analysis device 300 may be implemented to perform various analyses.

The image analysis device 300 may detect a lesion not only by using only the AI model 2 320 trained to detect a specific lesion (e.g., cancer), but may detect a region suspected of being a lesion by additionally using the AI model 1 310, and may perform more accurate and diverse analyses by integrating the two analysis results. A specific lesion to be detected in a medical image may be simply called a target lesion. The target lesion may be defined variously depending on the type of medical image or the task trained by the model. In the description, it is assumed that the image analysis device 300 detects a malicious lesion, i.e., cancer, in a medical image, such as Digital MMG or DBT.

AI model 1310 may be a model trained to detect a suspicious region in a medical image. That is, AI model 1 310 may detect a region suspected of being a lesion. For example, AI model 1310 may be trained to detect a region suspected of being a lesion or a region suspected of being likely to develop into a lesion in a medical image.

The training method of AI model 1310 may be various. AI model 1310 may be trained to detect a suspicious region using suspicious images and non-suspicious images. Labels of the training data may be attached variously. For example, the non-suspicious images are medical images that fall into a normal category and may be, for example, images that have a label of No visible abnormality. The non-suspicious images may include normal images or images with benign lesions, and the suspicious images may include images with malicious lesions, such as cancer, or suspicious-benign lesions. When a suspicious-benign lesion is detected, it is determined to be a benign lesion and a malicious lesion through additional examination (e.g., biopsy), so the suspicious-benign lesion may be called a biopsy benign.

The AI model 1 310 may output a score (simply referred to as a suspiciousness score) for a suspicious region detected in a medical image. Here, the suspiciousness score may be changed to another term. The suspiciousness score may be a value related to a confidence level at which the AI model 1 310 is certain that a suspicious region exists. The suspiciousness score may be defined as a value in a specific range (e.g., 0 to 100) or as a probability value between 0 and 1.

The AI model 2 320 may be a model trained to detect a specific lesion in a medical image. For example, the AI model 2 320 may be trained to detect breast cancer in a medical image, such as Digital MMG or DBT. If the AI model 2 320 is a model that receives a chest X-ray image as input, the AI model 2 320 may be trained to detect nodules, pneumothorax, pleural effusion, consolidation, cardiac hypertrophy, atelectasis, pneumoperitoneum, calcific degeneration, pulmonary fibrosis, mediastinal expansion, pulmonary tuberculosis, fractures, etc. The AI model 2 320 may be called a target lesion detection model or a cancer detection model.

A training method of AI model 2 320 may also vary. For example, if the AI model 2 320 is designed to detects cancer as a target lesion, lesion images with detected lesions and non-lesion images without detected lesions may be used as training data to reliably find the target lesion. That is, the AI model 2 320 may be trained to detect cancer from input images and output a score based on its findings, using cancer images in which cancer is detected (cancer images) and non-cancer images in which cancer is not detected (non-cancer images). Non-cancer images may include benign images or normal images.

The AI model 2 320 may output a score for any lesion (simply referred to as a lesion score) detected in a medical image. Here, the lesion score may also be changed to another term, such as a cancer score. The lesion score may be related to the confidence level at which the AI model 2 320 is certain that a target lesion exists in a medical image. This score reflects the model's confidence level in the presence of the target lesion within a medical image. The lesion score may be defined as a value within a specific range (e.g., 0 to 100) or as a probability value between 0 and 1.

The labels used for the training data may be attached variously. Referring to Table 1, the AI model 1 310 may be trained with images labeled as "Suspicious" labels and "Non-suspicious", while the AI model 2 320 may be trained with images labeled as "Cancer" labels and "Non-cancer".

(Table 1)

| | Label | |
|---|---|---|
| | AI model 1 suspicious region detection | AI model2 target lesion (cancer) detection |
| Malicious (e.g., cancer) | Suspicious | Cancer |
| suspicious-benign (biopsy benign) | | Non-cancer |
| benign (follow-up benign) | Non-suspicious | |
| normal | | |

An image suspected of having a target lesion may be labeled as "Suspicious", while an image not suspected of having a target lesion may be labeled as "Non-suspicious". The images suspected of having the target lesion may be an image including a malicious lesion, such as cancer, and an image including a suspicious-benign lesion. The image not suspected of having the target lesion may include an image including a general benign lesion and a normal image. Here, the suspicious-benign lesion may include a biopsy-confirmed benign lesion that is suspicious but have been deemed benign through a tissue examination. The general benign lesion may include a follow-up benign that is determined to be a benign lesion as a result of a follow-up observation.

If the target lesion is cancer, the image including the target lesion may be annotated with a "Cancer" label, and the image not including the target lesion may be annotated with a "Non-cancer" label. The image that does not include the target lesion may include a normal image, an image including a benign lesion, and an image including a suspicious-benign lesion.

In this manner, the AI model 2 320 learns by using a medical image that includes the target lesion as positive data to find the target lesion (cancer). The AI model 1 310 also learns by using a medical image that does not include the target lesion but is suspected of being the target lesion as positive data. Therefore, through the AI model 1 310, a region that the AI model 2 320 may miss may be found with a conservative criterion.

The integrator 330 of the image analysis device 300 may calculate scores for regions of the medical image based on the suspiciousness score and lesion score obtained from the AI model 1 310 and the AI model 2 320 and determines the class to which the corresponding region belongs based on the score. The regions of the medical image may be classified into any one of normal, indeterminate, and abnormal depending on the score. The class name may be adjusted as needed. Here, the indeterminate region is an ambiguous region that the AI model cannot determine whether the region is normal or abnormal, requiring further judgment from the user. The abnormal region may be a lesion region, and they may be used interchangeably in the description. The score calculated based on the suspiciousness score and the lesion score may be referred to as a rearrangement score. To provide a comprehensive reading of the analysis results of the AI model through the suspiciousness score and the lesion score, the integrator 330 may adjust the suspiciousness score and the lesion score into a single rearrangement score and analyze the medical image based on the rearrangement score. For example, if the region has a suspiciousness score of 80 and the lesion score of 2, the two scores may be adjusted into a single score, and which will then be used to classify the region as normal, indeterminate, or abnormal. The highest rearrangement score analyzed in the medical image may be used as the abnormality score of the medical image.

The integrator 330 may determine a medical image as a normal case if the entire regions are determined to be normal. The integrator 330 may determine a medical image as an abnormal case if the medical image includes either an indeterminate region or an abnormal region. An image including an indeterminate region may be called an indeterminate case, an image including a lesion may be called an abnormal case, and both an indeterminate region and a lesion region may be detected in one medical image.

The image analysis device 300 may detect the indeterminate region, allowing users to review a suspicious region, which is not a lesion though, without missing. A medical image that does not include any indeterminate regions can be confidently classified as a normal case.

The calculation of the rearrangement score can be done using various methods. For example, the integrator 330 may calculate the rearrangement score $Score_{Rearrangement}$, based on the abnormality score $Score_{suspicious}$ and the lesion score $Score_{cancer}$, as shown in Equation 1. Given that the lesion score is considered more significant than the abnormality score, a weight w1 for the abnormality score may be set to a value less than 1 (e.g., 0.5). Also, a weight w2 (e.g., 0.5) may be applied to the product of the lesion score and the abnormality score to compute an arithmetic mean.

(Equation 1)

$$Score_{Rearrangement} = (Score_{Cancer} * Score_{Suspicious}^{w1})^{w2}$$

The integrator 330 may set a first threshold value that distinguishes between normal and indeterminate and a second threshold value that distinguishes between indeterminate and abnormal (e.g., cancer) and determine a class corresponding to the rearrangement score based on the threshold values. The first threshold value is a value that distinguishes between normal and abnormal, and the first threshold value may be determined so that a score smaller than the first threshold value is determined as normal. The second threshold value may be determined so that a score larger than the second threshold value is determined as a target lesion (e.g., cancer).

The first threshold value and the second threshold value may be determined based on the rearrangement score. Specifically, the first and second thresholds for the rearrangement score may be determined to achieve desired sensitivity and specificity of the AI model. For example, the rearrangement score may range from 0 to 100, the first threshold may be set at 2, and the second threshold may be set at 10, but the threshold may be adjusted according to various conditions including sensitivity and specificity.

There may be various methods for determining the threshold value. For example, the integrator 330 may create a linearly increasing value by shifting the overall value according to a specific sensitivity and specificity through linear movement, thereby integrating the abnormality score and the lesion score with the same standard. Alternatively, the threshold value may be determined experimentally or statistically.

The image analysis device 300 may generate a final analysis result for the medical image based on the analysis results of the AI model 1 310 and the AI model 2 320. Here, the final analysis result may be an overall analysis result including analysis results of an AI model for a medical image and results obtained by comprehensively analyzing them.

The image analysis device 300 may transmit at least a portion of the final analysis result to a designated device. For example, the final analysis result may include results obtained from analyses performed on the medical image, for example, normal case information indicating whether it is a normal case, indeterminate region information, abnormal (lesion) region information, abnormality score, medical indicator, etc. In addition, the final analysis result may include rearrangement scores for regions of the medical image. When the image analysis device 300 performs an automatic report creation function, an automatically generated report for a normal case may be included in the final analysis result. The final analysis result may include data for providing an analysis result providing image (e.g., SC image, GSPS, etc.), a worklist, a report, etc. described in the disclosure, and at least a portion of the final analysis result may be transmitted to a designated device. The image analysis device 300 may provide the analysis result for the medical image in formats, such as DICOM's secondary capture (SC), grayscale softcopy presentation state (GSPS), and structured report (SR). The analysis result for the medical image may be stored in the image storage device 200 and displayed in the user terminal 100 linked to the image storage device 200. If the medical image is a DICOM image, metadata of the image may be stored in public tags and private tags. Information on the medical image is recorded in the Public tag according to a file structure specified in the DICOM standard. Since the private tag may be freely used when a medical device company wants to add information not included in the public tag to the DICOM image, the analysis result for the medical image may be recorded in the private tag. Meanwhile, the information included in the private tag may be defined variously. For example, information indicating a normal case, for example, "No visible abnormality", may be added to the private tag of a normal case, and information indicating not a normal case may be added to the remaining cases, or information on a classification result may not be added.

The viewer 110 run on the user terminal 100 may provide the analysis result for the medical image. The viewer 110 may display the analysis result generated in the DICOM format on a screen.

In order for the user to quickly and accurately recognize the analysis result of the medical image, the user terminal 100 may provide the analysis results for medical images depending on whether they are normal cases and may also provide indeterminate regions suspected of being lesions as well as lesion regions, such as cancer.

A worklist may include a study case list, each study case including at least one medical image for image reading task. The worklist, which presents a list of medical images for user reading, may provide medical images distinguished according to the analysis results. The worklist may indicate that a medical image is a normal case, indicate that there is an indeterminate region in the medical image, or indicate that there is a lesion in the medical image. Meanwhile, medical images classified as normal cases may be excluded from the worklist and stored in a separate folder. In this case, the user may separately check only the medical image classified as a normal case through the folder. The SC image and GSPS that provide the analysis results may display a graphical indicator that visually represents a classification result of the medical image. For example, an SC image for a normal case may display a graphical indicator, such as an "N" icon, indicating that it is a normal case so that the user may quickly check the normal case. "N" may refer to No visible abnormality or Normal. Meanwhile, when providing a preview image (a thumbnail image) of the medical image in the PACS worklist, a preview image for a normal case may display a graphical indicator indicating that it is a normal case so that the user may quickly check the normal case. In addition to the normal case, a designated graphical indicator may be displayed on the SC image or the preview image so that the user may quickly check a medical image including an indeterminate region.

The SC image of the medical image in which a lesion is detected may display a lesion region through various methods (Color Heatmap, Single Colormap, Contour map, Combined map, etc.). In addition, the SC image of the medical image in which an indeterminate region is detected may display the indeterminate region, and may display the indeterminate region to be distinguished from the lesion region. For example, the SC image may indicate the indeterminate region as a white square or white circle, or as an angle symbol, such as parentheses, and may also display the indeterminate region variously that may not interfere with the user's reading. The display method of the lesion region and the indeterminate region may be changed through configuration.

Medical images classified as normal cases may be set to automatically generate a report. The user may approve the automatically generated report to complete the reading task for the corresponding normal case.

In this manner, the image analysis device 300 may classify the medical image into a normal case or an abnormal case including an indeterminate region or a lesion region and may provide the medical image or analysis result differently according to the classification result. Therefore, the user may reduce the time required for reading and creating a report for the normal case. In addition, the user may interpret the indeterminate region that the AI model determines to be suspicious but not a target lesion without missing.

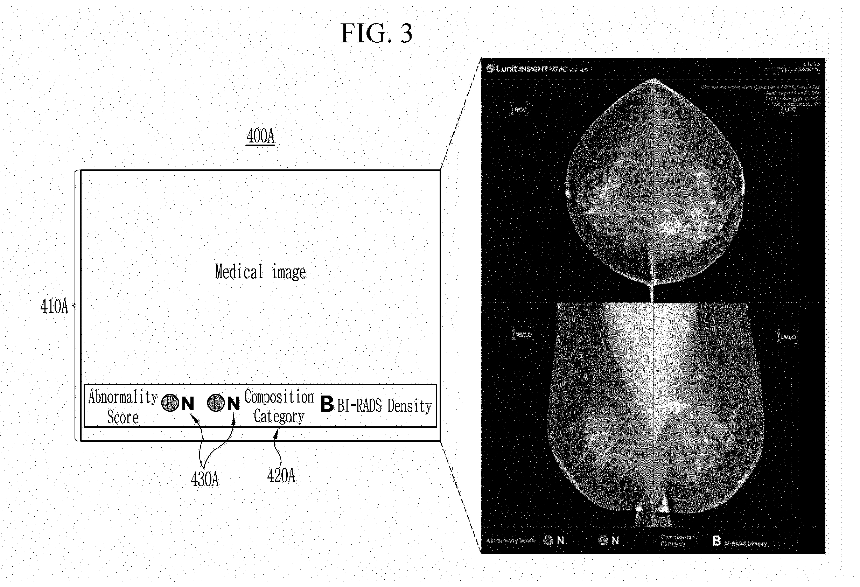

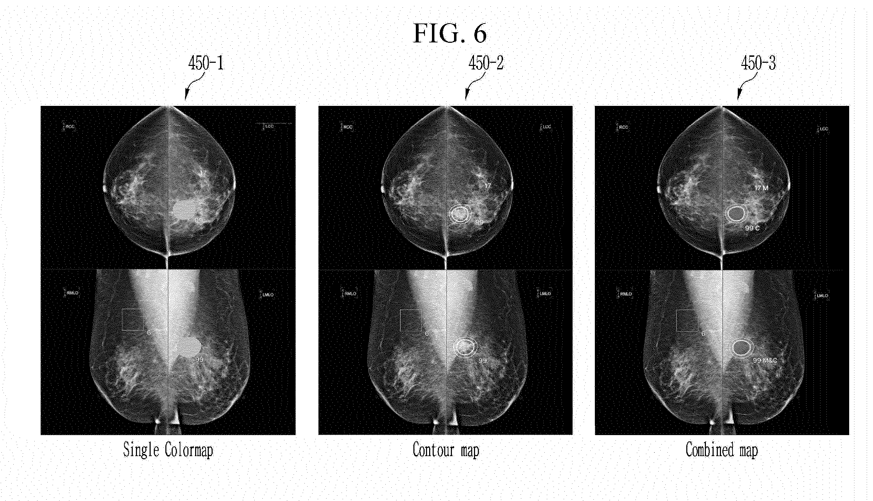

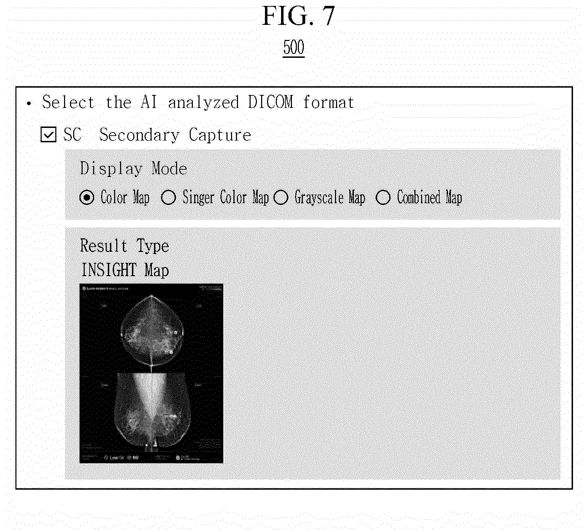

FIGS. 3 to 5 are examples of images that provide analysis results of medical images according to an exemplary embodiment, FIG. 6 is an example of a method for displaying a lesion region according to an exemplary embodiment, and FIG. 7 is an example of an interface for setting an analysis result display method according to an exemplary embodiment.

Referring to FIG. 3, an analysis result of a medical image may be provided in a DICOM SC format.

An SC image 400A for a normal case may include an image region 410A in which a medical image is displayed and an information region 420A in which an analysis result of the medical image is displayed. Information displayed in the information region 420A may be defined variously. The information region 420A may display an abnormality score or may display a medical indicator specified according to the type of an analysis target and/or the medical image. The abnormality score may be a rearrangement score generated based on scores obtained from the AI model 1310 and the AI model 2 320. If a plurality of regions are detected in the medical image, the highest score among the rearrangement scores of the regions may be displayed as the abnormality score.

In the SC image 400A, the abnormality score does not necessarily have to be displayed as a number and may indicate the classification result of normal, indeterminate, or abnormal or may indicate a level, such as lower (Low) than a specified threshold value, higher (High) than a specified threshold value, etc. The type of information to be displayed in the information region 420A may be determined according to the abnormality score or the classification result. For example, depending on the abnormality score or classification result, a value to be displayed in the information region 420A among the score, classification result, and level may be selected or combined. For example, in the case of a normal case, a graphical indicator that intuitively indicates that it is a normal case may be displayed in the information region 420A instead of the score. For a medical image that is not a normal case but has a low abnormality score, the score and the low level may be displayed together in the information region 420A, and for a medical image that has a high abnormality score, only the score may be displayed or the score and the high level may be displayed together. In addition, a graphical indicator that indicates an indeterminate case or an abnormal case may be displayed.

The SC image 400A for a normal case may display a graphical indicator that indicates that the medical image is a normal case. The position and display method of the graphical indicator may be defined variously. For example, the graphical indicator that indicates a normal case may be displayed as an "N" icon. "N" may refer to No visible abnormality or Normal. For example, instead of an abnormality score in the information region 420A, a graphical indicator 430A that intuitively indicates a normal case may be displayed.

If the medical image is a mammography image, such as Digital MMG or DBT, the information region 420A may display the analysis results of the left and right breasts separately or display the analysis results of the left and right breasts collectively. For example, the abnormality score displayed in the information region 420A may be displayed separately for the left breast and the right breast. At this time, the abnormality score may be displayed according to the scores of the left and right breasts respectively, and in the case of normal, the graphical indicator "N" may be displayed. The graphical indicator and the score may be displayed together. Meanwhile, the abnormality score displayed in the information region 420A may be displayed as a single score that integrates the scores of the left and right breasts.

The information region 420A may display information related to breast density analyzed by the AI model. The information region 420A may display a density score analyzed by the AI model. The breast density may be provided as a Composition Category according to Breast Imaging Reporting & Data System (BI-RADS). According to BI-RADIS (5th edition), A Category may refer to Almost Entirely fatty, B Category may refer to Scattered Fibroglandular Densities, C Category may refer to Heterogeneously dense, and D Category may refer to Extremely Dense. The density score may be expressed as a value between 0 and 10 points, for example, and a value closer to 10 points may indicate denser breast.

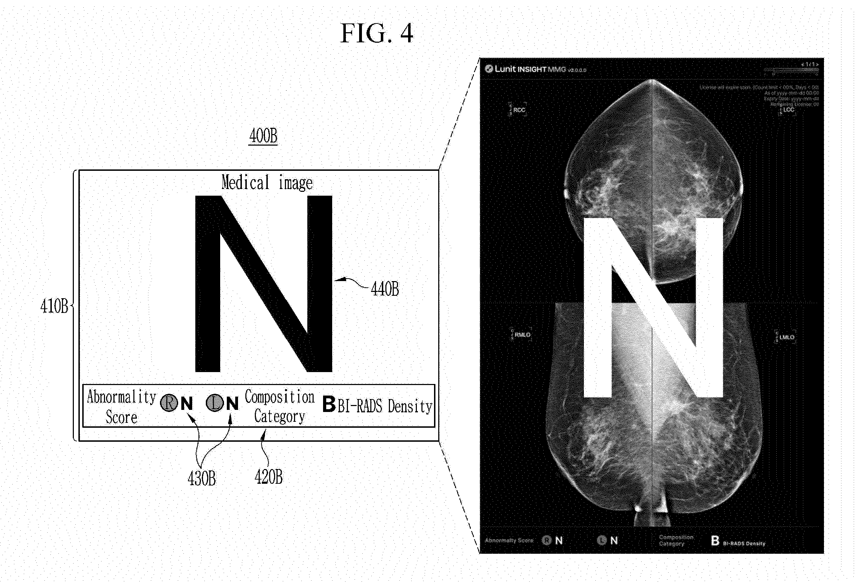

Referring to FIG. 4, an SC image 400B for a normal case may include an image region 410B in which a medical image is displayed and an information region 420B in which an analysis result of the medical image is displayed. The information region 420B may display information related to an abnormality score and information related to breast density, similar to FIG. 3.

At this time, the SC image 400B for the normal case may display a graphical indicator indicating that the medical image is a normal case. The graphical indicator may be displayed as an "N" icon.

Similar to FIG. 3, a graphical indicator 430B indicating that the medical image is a normal case may be displayed in the information region 420B. A graphical indicator 440B indicating that the medical image is a normal case may be displayed in the image region 410B. The graphical indicator 440B may be displayed on the medical image. The graphical indicator 440B may be displayed at all times, may be displayed for a certain period of time (e.g., 5 seconds after viewing) and then disappear, and may be displayed or disappear by a user input (e.g., click) using an input unit, such as a microphone, mouse, or keyboard. The user may quickly recognize that it is a normal case by looking at the graphical indicator 430B displayed in the information region 420B, and may more quickly recognize that it is a normal case by looking at the graphical indicator 440B displayed in the image region 410B.

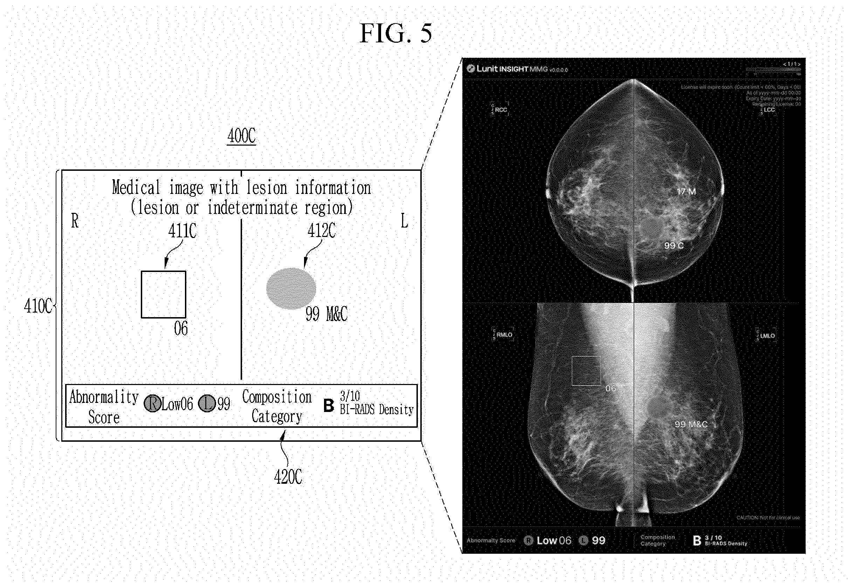

Referring to FIG. 5, an SC image 400C of a medical image that is not a normal case may include an image region 410C in which a medical image is displayed and an information region 420C in which an analysis result of the medical image is displayed. The information region 420C may display information related to an abnormality score and information related to breast density, as described in FIG. 3.

For example, if an indeterminate region is detected as a result of analysis of the AI model for the right breast image, the SC image 400C may display an indicator 411C indicating the indeterminate region and may display a score (e.g., 06) together with the indicator 411C. The indicator 411C indicating the indeterminate region may be displayed as a square or may be displayed as a circle or angle symbol, such as parentheses. The score displayed together with the indicator 411C is a rearrangement score for the indeterminate region, and if this rearrangement score is the highest rearrangement score, it may be displayed in the information region 420C. For example, a suspicious-benign lesion may be detected as the indeterminate region. The indicator indicating the indeterminate region may be provided in a form that does not obscure the indeterminate region as much as possible, but indicates that it is a region that an interpreter should read carefully and may be displayed as an achromatic line, such as white or black.

For example, if a lesion is detected as a result of the analysis of the AI model for the left breast image, the SC image 400C may display an indicator 412C indicating the lesion region (abnormal region) and may display a score (e.g., 99) and a lesion type (e.g., Mass, Calcification, etc.) together with the indicator 412C. The lesion region may be displayed as a color heatmap and may be displayed in various other ways. The color heatmap may be an indicator that displays a color matching each pixel score of the medical image on the image. The score displayed together with the indicator 412C may be a rearrangement score for the lesion region, and the highest rearrangement score may be displayed in the information region 420C.

The abnormality score displayed in the information region 420C may be displayed to be distinguished between the left breast and the right breast. The abnormality score of the right breast may indicate a rearrangement score (e.g., 06) for the indeterminate region, and a low (Low) level may be displayed together with the score, so that the user may intuitively know that the abnormality score is low. In a case in which a plurality of lesion regions are detected in the left breast, the abnormality score of the left breast may be represented by the highest rearrangement score (e.g., 99). In addition, the information region 420C may display a graphical indicator indicating that an indeterminate region is detected in the right breast image or a graphical indicator indicating that a lesion is detected in the left breast image.

In addition, the information region 420C may display breast density-related information analyzed by the AI model and may display, for example, categories and density scores classified according to BI-RADIS.

Since the related art image analysis device uses only the AI model that detects a region in which a lesion exists, the SC image may display only the lesion region, such as the left breast image. Since the related art image analysis device does not display any region in which a lesion score is lower than a threshold value, the user has the inconvenience of having to recheck the entire image to find a suspicious region that does not appear to be cancer but cannot be considered normal. In contrast, the image analysis device 300 of the disclosure may detect not only a lesion region but also an indeterminate region. Accordingly, the SC image 400C may display a suspicious region, but not a lesion region, like the right breast image, as an indeterminate region 411C,

At this time, the indeterminate region 411C may be displayed to be different from the lesion region 412C. Accordingly, the user may carefully interpret not only the lesion region 412C, such as cancer, but also the indeterminate region 411C, such as a suspicious-benign lesion.

Referring to FIG. 6, the lesion region may be displayed as a single color map 450-1, a contour map 450-2, a combined map 450-3 of a color map and a contour map, in addition to a color heatmap.

The single color map is a method of displaying a lesion region with a single color, the contour map is a method of displaying the border of a lesion region with a line, and the combined map is a method of displaying a lesion region using both color and contour.

Referring to FIG. 7, the user terminal 100 may display a setting interface 500 that may set a method of displaying an analysis result of the AI model. In the setting interface 500, the user may select a display mode of the color map, the single color map, a gray scale map (the contour map), and the combined map to display the analysis result in a desired format.

In this manner, an analysis result of a medical image may be provided as an SC image and may also be provided as a GSPS. When the analysis result is provided as a GSPS, the analysis result including a graphical indicator indicating a normal case, an indeterminate region, a lesion region, etc. may be overlaid to be displayed on the original medical image. Here, the GSPS may display the lesion region as the gray scale map (the contour map).

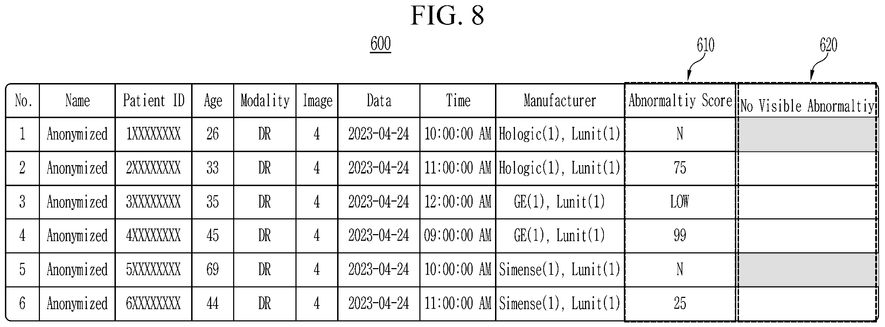

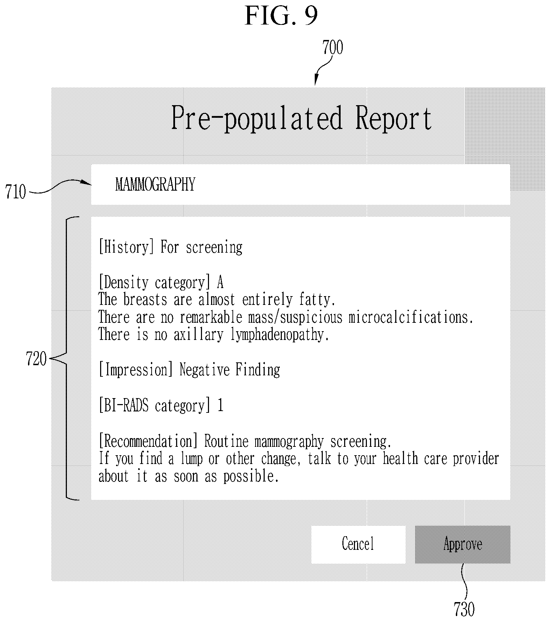

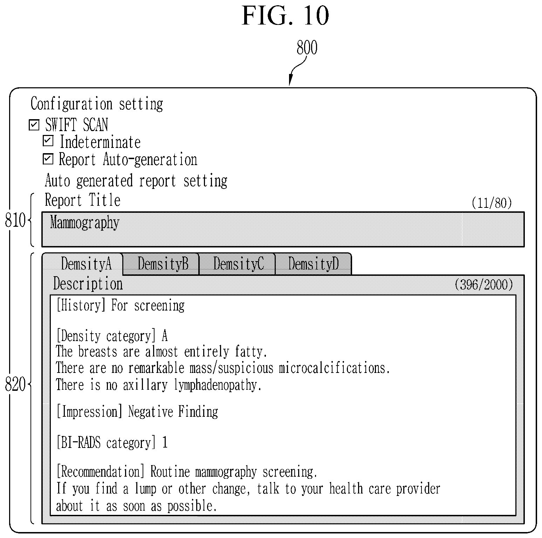

FIG. 8 is an example of a worklist according to an exemplary embodiment, FIG. 9 is an example of an automatically generated report according to an exemplary embodiment, and FIG. 10 is an example of a report setting interface according to an exemplary embodiment.

Referring to FIG. 8, the viewer 110 executed on the user terminal 100 may provide a worklist 600 in conjunction with the image storage device 200. The worklist 600 may display a list of images that the user has to interpret in a table format along with key information. The worklist does not necessarily need to be included in the viewer 110 and may be installed as a separate program. The information displayed in the worklist may be provided by the viewer 110 or the image storage device 200, but for convenience of description, it may be described that the viewer 110 provides the information.

The viewer 110 may display an analysis result analyzed by the image analysis device 300 for a medical image in designated columns 610 and 620 of the worklist 600 by interworking with the image storage device 200. The analysis result displayed in the worklist may be set variously. For example, the abnormality score of each image may be written in the column 610, and the normal case information may be written in the "No Visible Abnormality" column 620. For example, the viewer 110 may check whether a case is normal based on the information included in the DICOM private tag of the medical image and may display the normal case in various manners to distinguish the normal case from the other cases in the worklist.

The viewer 110 may display the images included in the worklist in a distinct manner according to the priority determined based on the analysis result. In addition, the viewer 110 may sort and display an image list of the worklist according to the priority determined based on the analysis result. Through this, the user may identify the normal case and determine work priority and set the priority to interpret an abnormal case (an indeterminate case, an abnormal case) before the normal case. In addition, the user may spend more time on the abnormal case than the normal case to perform the reading task.

The worklist 600 may display an indicator indicating a normal case in the designated column 620 so that the normal case may be quickly identified. For example, the worklist 600 may display a normal case indicator (e.g., a flag display, a highlight display, or a "No Visible Abnormality" display, etc.) in the designated column 620 of a specific medical image and may provide a function for sorting or extracting the normal case for normal case reading. The normal case indicator may be displayed as various visual components distinguished by text, shapes, colors, etc. Through this, the user may check whether the corresponding image is a normal case through the normal case indicator even without opening the medical image of the worklist 600.

Meanwhile, the worklist 600 may not display a medical image classified as a normal case by the AI model or may provide a function of storing only normal cases in a separate folder so that the user may collectively view them.

A report explaining the analysis result may be automatically generated for the medical image classified as a normal case. The worklist 600 may display an indicator indicating that there is a report for the normal case. For example, for the normal case, the worklist 600 may display a button for checking a pre-populated report in a designated column (e.g., the "No Visible Abnormality" column) or may activate a function of checking the report (e.g., a report pop-up function when a designated region is clicked). The report may be displayed on the screen in response to a designated user input (e.g., a click) using an input unit, such as a microphone, mouse, or keyboard. The user may recognize that a report requiring user confirmation has been generated through an indicator indicating report generation. Even if the user does not open the medical image of the worklist 600, the user may confirm the automatically generated report and complete the work for the corresponding case by approving the report. The automatically generated report may be modified by the user.

Referring to FIG. 9, the user terminal 100 may display a pre-populated report 700 for a normal case. The report 700 may include phrases preset by the user and may include phrases corresponding to the analysis result of the medical image. For example, if the medical image is a mammography image, phrases according to breast density may be set in advance, and phrases may be inserted into the report according to the analysis result.

The report 700 may be generated using a designated template, may be structured, for example, into a region 710 in which report information (e.g., MAMMOGRAPHY) is described and a description region 720, and may include an approval button 730. In addition, the report template may be structured to include information required according to the report type. The button type may be changed variously. Text displayed in the image information region 720 may be created and modified by the user. The user may click the approval button 730 to complete the work for the corresponding case.

In the description region 720, set phrases may be written according to the analysis result. For example, in the case of a medical image in which breast density is analyzed as category A, A is written in a [Density category] item, and phrases corresponding to the category A (e.g., "the breasts are almost entirely fatty. There are no remarkable mass/suspicious microcalcifications. There is no axillary lymphadenopathy."), among phrases set by category, may be inserted. Depending on the analysis result, 1 is written in a [BI-RADS category] item, and set phrases may be written in items, such as [Impression] and [Recommendation], set by the user.

Referring to FIG. 10, the user terminal 100 may display a report setting interface 800 in which the user may create phrases to be written in an automatically generated report. The report setting interface 800 may be provided by a designated device, for example, a report generating device, and the report generating device may be implemented in the image analysis device 300 or implemented to interwork with the image analysis device 300.

The report setting interface 800 may display a layout in which text may be input and may include a region 810 in which report information, such as a report title, may be input, and a region 820 in which a description according to an analysis result may be input. The report setting interface 800 may provide a layout in which the user may input phrases suitable for the analysis result. For example, in the case in which a report is to be created based on breast density analyzed from a mammography image, the report setting interface 800 may provide a window in which the user may write desired phrases for each breast density category (Density A, Density B, Density C, and Density D) and may store the report contents created by the user by category. The user may freely change the report contents through the report setting interface 800.

Thereafter, if the medical image is classified as a normal case, a report may be automatically created with phrases set by the user in the report setting interface 800. For example, in the case of a normal case in which breast density is analyzed as category A, the report 700 of FIG. 9 may be created with phrases created for Density A in the report setting interface 800.

In this manner, through the automatic report creation function for a normal case, the user may reduce a report creation time and complete the reading task for the normal case by approving the automatically generated report. In addition, even for the normal case, such as a mammography image, the report contents may need to be different depending on the analysis result. According to the disclosure, since the user may previously set the phrases to be written in the report according to the breast density in the report setting interface 800, the inconvenience of having to check the analysis result of each image and then create the reading contents to correspond to the analysis result may be resolved.

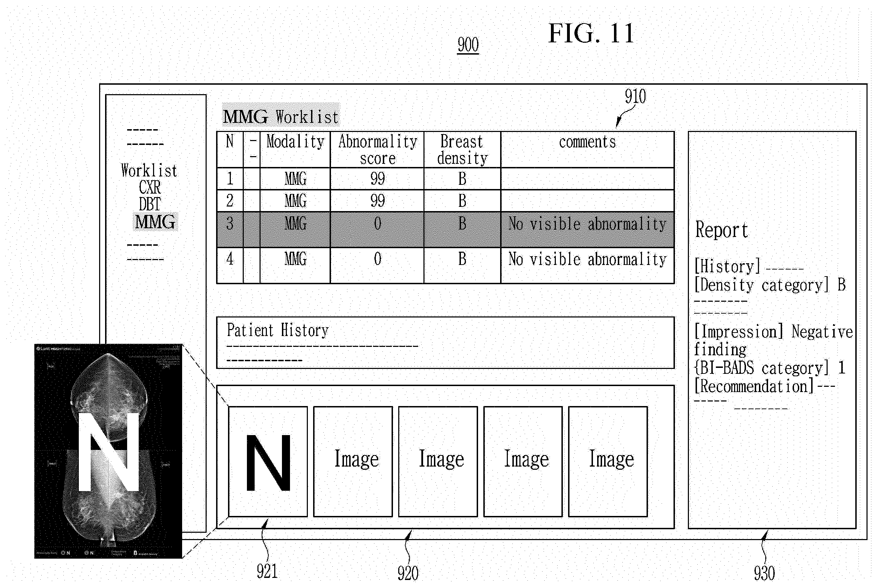

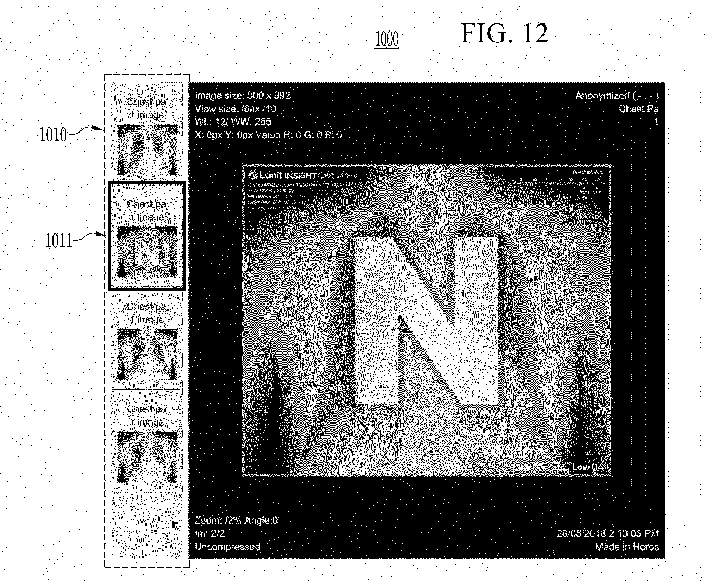

FIG. 11 is an example of a worklist that provides a preview image according to an exemplary embodiment, and FIG. 12 is an example of an image providing interface for a normal case according to an exemplary embodiment.

Referring to FIG. 11, the user terminal 100 may display a worklist 900. A layout of the worklist 900 may vary and may include a worklist table 910 that displays a list of images and a preview region 920 that shows images to be interpreted in advance. In addition, the worklist 900 may include a report region 930 that shows a report created for an image.

The worklist table 910 may display an analysis result for a medical image, as shown in FIG. 8, and the analysis result may vary depending on an image modality. The worklist table 910 may display, for example, an abnormality score, normal case information (e.g., "No Visible Abnormality" indication), and an analysis result, such as breast density.

The preview region 920 may provide a preview image included in the worklist in the form of a thumbnail, etc. A preview image (thumbnail image) 921 may include a graphical indicator indicating the analysis results for the corresponding medical image. For example, the preview image 921 may include a graphical indicator indicating a normal case, for example, an "N" icon. The graphical indicator indicating a normal case may be defined variously. Even if the user does not open and check the medical image, the user may identify the normal case through the normal case indicator in the worklist table 910 and also identify the normal case through the graphical indicator displayed in the preview image. In particular, the preview image 921 may provide the analysis result in advance through the graphical indicator that is visually emphasized rather than a normal case indicator displayed as text in the worklist table 910, so that an reading speed may significantly increase.

The report region 930 may display an automatically generated report for a normal case if the medical image is the normal case. The report region 930 may include a function of modifying or approving the automatically generated report.

Referring to FIG. 12, the analysis result of the medical image may be provided as an SC image, etc. or may be displayed in the preview image. An interface 1000 that displays the medical image may display the selected medical image and may provide a preview region 1010 that provides viewable medical images in a small size. The preview region 1010 may display a graphical indicator that allows intuitive recognition of the analysis result for the medical image. For example, a preview image 1011 may display a graphical indicator, for example, an "N" icon, indicating that it is a normal case.

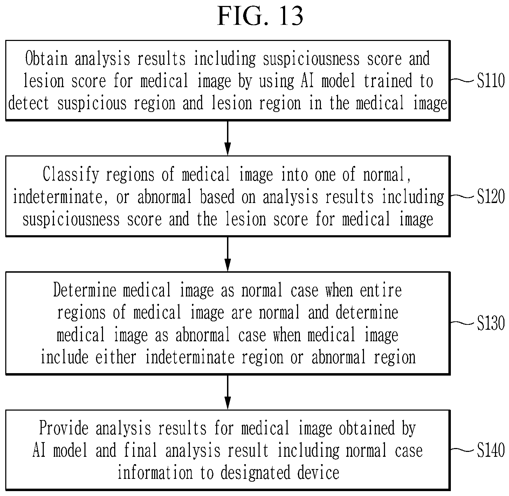

FIG. 13 is a flowchart of an image analysis method according to an exemplary embodiment.

Referring to FIG. 13, the image analysis device 300 obtains analysis results including a suspiciousness score and a lesion score for a medical image by using an AI model trained to detect a suspicious region and a lesion region in the medical image (S110). The image analysis device 300 may detect a suspicious region in the medical image and also use the AI model trained to detect a target lesion (e.g., cancer) in the medical image. The AI model that detects a target lesion (e.g., cancer) in the medical image may be further trained to detect a suspicious region. The image analysis device 300 may obtain analysis results including a suspiciousness score that is certain that a suspicious lesion exists and a lesion score that is certain that a lesion exists in each region.

The image analysis device 300 classifies regions of the medical image into one of normal, indeterminate, or abnormal based on the analysis results including the suspiciousness score and the lesion score for the medical image (S120). The image analysis device 300 may calculate a rearrangement score based on the suspiciousness score and the lesion score and determine a class (normal, indeterminate, abnormal) corresponding to the rearrangement score using a first threshold value that distinguishes between normal and indeterminate and a second threshold value that distinguishes between indeterminate and abnormal. The specific class name may vary, and a method used by the image analysis device 300 to calculate the rearrangement score based on the suspiciousness score and the lesion score may include a method of calculating the rearrangement score by applying a weight to the suspiciousness score and the lesion score.

The image analysis device 300 determines the medical image as a normal case when the entire regions of the medical image are normal and determines the medical image as an abnormal case when the medical image includes either an indeterminate region or an abnormal region (S130). The indeterminate region may refer to a region that cannot be considered a lesion (e.g., cancer) but also cannot be considered normal. The abnormal region may refer to a lesion region.

The image analysis device 300 provides the analysis results for the medical image obtained by the AI model and a final analysis result including normal case information to a designated device (S140). The analysis results for the medical image may include indeterminate region information, lesion information, abnormality score, a medical indicator, etc. Meanwhile, the final analysis result may include an automatically generated report for the normal case. The final analysis result for the medical image may be generated in a DICOM format and transmitted to the image storage device 200. The final analysis result for the medical image may be stored in the image storage device 200, which is, for example, a PACS server/DB, and may be displayed through the viewer 110 in the user terminal 100 interworking with the image storage device 200. The final analysis result for the medical image is generated in a standard established to enable information compatibility between medical information systems, for example, an HL7 message format, and may be transmitted to a medical information system, such as an EMR system or EHR system.

A medical image classified as a normal case may be handled differently from an abnormal case in the medical image system 10.

An SC image or GSPS that provides the analysis result for a normal case may include a graphical indicator (e.g.,

an "N" icon) indicating that it is a normal case. A preview image (a thumbnail image) for a normal case may include a graphical indicator (e.g., an "N" icon) indicating that it is a normal case.

When a normal case is assigned to a worklist, the worklist may display an indicator (e.g., a flag, a highlight, or a "No Visible Abnormality" indicator) indicating that it is a normal case. Alternatively, the medical image of the normal case may not be assigned to the worklist or may not be displayed in the worklist. Alternatively, the medical image of the normal case may be stored in a separate folder so that the user may collect and check only the normal cases.

A report may be automatically generated for the medical image of the normal case. The worklist may display an indicator indicating that a report is automatically generated for the normal case. The user may recognize the report creation through the indicator indicating the report creation. Even if the user does not open the medical image in the worklist, the user may check the automatically generated report and complete the work for the corresponding case by approving the report. The automatically generated report may be modified by the user.

Meanwhile, a medical image including an indeterminate region or a lesion region (abnormal region) may be classified as an abnormal case. In the case of the abnormal case, the indeterminate region and the lesion region may be displayed in different forms in an image providing analysis results (e.g., an SC image).

The indicator indicating the lesion region may be displayed as a color heat map, a single color map, a contour map, a combined map of a color map and a contour map, etc.

Unlike the lesion region, the indicator indicating the indeterminate region may be provided in a form that does not obscure the indeterminate region as much as possible, but indicates that it is a region that the user should pay attention to in order to interpret. For example, the indicator indicating the indeterminate region may specify the region in a shape, such as a square, a circle, an angle symbol, such as parentheses, and the border of the shape may be set to an achromatic color, such as white or black.

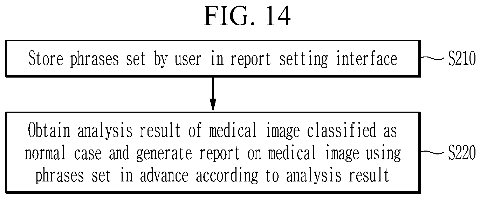

FIG. 14 is a flowchart of a method for automatically generating a report according to an exemplary embodiment.

Referring to FIG. 14, a report generating device stores phrases set by the user in the report setting interface (S210). The image analysis device 300 may have a report creation function, but the report creation function may be implemented in a device that may access an analysis result of a medical image, and the report may be automatically generated by an AI model trained to generate a report. The report generating device may receive phrases to be inserted into the report according to an analysis result of a normal case through the report setting interface. Meanwhile, when the report has to be generated differently according to an analysis result even in a normal case, such as a mammography image, the report setting interface may provide a window in which the user may write phrases for each breast density category (Density A, Density B, Density C, and Density D) and may receive report contents from the user by category.

The report generating device obtains the analysis result of the medical image classified as a normal case and generates a report on the medical image using the phrases set in advance according to the analysis result (S220). The report generating device may check the analysis result of medical image, for example, the breast density category, and generate a report using the phrases set in the category.

The automatically generated report for the medical image of the normal case may be provided through a worklist, and a user-approved report may be stored in a designated device. The worklist may display an indicator indicating that a report has been automatically generated for a normal case. The worklist may display a button for checking a pre-generated report for the normal case or may activate a function of checking the report. The report may be linked to the worklist so that the report is displayed on the screen in response to a designated user input (e.g., click) using an input unit, such as a microphone, mouse, or keyboard.

The automatically generated report for the medical image of the normal case may be stored in a medical information system, such as an EMR. Through this, a medical professional may access the EMR system, check a pre-generated report, and approve the report.

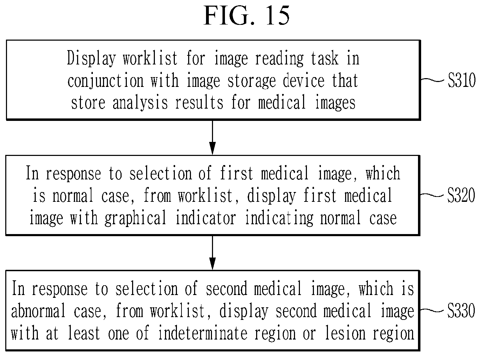

FIG. 15 is a flowchart of a method for providing an analysis result according to an exemplary embodiment.

Referring to FIG. 15, the viewer 110 run on the user terminal 100 displays a worklist for image reading task in conjunction with the image storage device 200 that stores analysis results for medical images (S310). The viewer 110 displays the analysis result of each image analyzed by the image analysis device 300 in a designated column of the worklist, and the analysis result may include an abnormality score and whether it is a normal case. The worklist may provide a function of not displaying or hiding the medical image of the normal case. Alternatively, the normal case may not be assigned to the worklist for reading. The worklist may display an indicator indicating a normal case (e.g., a flag display, a highlight display, or a "No Visible Abnormality" display) in a table-type image list. The worklist may display an indicator indicating that there is a report on the normal case. The worklist may display an indicator indicating that it is a normal case in a preview image (a thumbnail image) of the medical image. For example, the preview image may include an "N" icon indicating that it is a normal case.

In response to a selection of a first medical image, which is a normal case, from the worklist, the viewer 110 displays the first image with a graphical indicator indicating the normal case (S320). For example, an SC image for the normal case may display a graphical indicator indicating that it is a normal case so that the user may quickly recognize the normal case. The normal case indicator may be displayed variously in the image. For example, as in FIG. 3 or FIG. 4, the

**EP 4 564 364 A1**

normal case indicators (e.g., the "N" icon) 430A and 430B may be displayed instead of a score in the information region 420A and 420B that provides the analysis results. Alternatively, as shown in FIG. 4, the graphical indicator 440B may be displayed in the image region 410B in which a medical image is displayed.

In response to a selection of a second medical image, which is an abnormal case, from the worklist, the viewer 110 displays the second medical image with at least one of an indeterminate region or a lesion region (S330). The viewer 110 may display the indeterminate region and the lesion region in different forms so that the indeterminate region and the lesion region may be distinguished from each other. The indeterminate region may be a region that is not a lesion but is determined to be suspicious by the AI model. The lesion region may be a region determined to be abnormal by the AI model and may be, for example, a cancer region.

The viewer 110 is implemented as a computer program stored in a computer-readable recording medium and includes instructions that are executed by a processor. The computer program may include instructions that cause the executing processor to display a worklist including a list of images for image reading task by interworking with an image storage device that stores analysis results for medical images and to display analysis results of the medical image when a medical image is selected from the worklist. The computer program may include instructions to display an indicator indicating that a specific medical image is a normal case in the image list, display an indicator indicating that a specific medical image is a normal case in the preview image of the specific medical image, or display an indicator indicating that a report has been generated in advance for the specific medical image, when the specific medical image is confirmed to be a normal case based on normal case information included in the analysis results of the medical images.

The terminal or device 100, 200, and 300 constituting the medical imaging system 10 of the disclosure may include one or more processors, a memory for loading a computer program executed by the processor, a storage device storing the computer program and various data, and a communication interface. In addition, the terminal or device 100, 200, and 300 may further include various components. The processor may be a variety of processors that process instructions included in the computer program and may include at least one of a central processing unit (CPU), a micro processing unit (MPU), a micro controller unit (MCU), a graphics processing unit (GPU), or any other type of processor well known in the art of the disclosure. The memory stores various data, instructions, and/or information. The memory may be implemented to store instructions so that the instructions described to execute the operations of the disclosure are processed by the processor. The memory may be, for example, a read only memory (ROM), a random access memory (RAM), etc. The storage device may non-temporarily store a computer program and various data. The storage device may be configured to include a non-volatile memory, such as a read only memory (ROM), an erasable programmable ROM (EPROM), an electrically erasable programmable ROM (EEPROM), a flash memory, a hard disk, a removable disk, or any form of a computer-readable recording medium well known in the art to which the disclosure pertains. The communication interface may be a wired/wireless communication module that supports wired/wireless communication. The computer program includes instructions executed by the processor and is stored in a non-transitory computer readable storage medium, and the instructions cause the processor to execute the operations of the disclosure.

The exemplary embodiments of the disclosure may not necessarily be implemented only through the foregoing devices and methods but may also be implemented through a program for realizing functions corresponding to the configurations of the exemplary embodiments of the disclosure, a recording medium including the program, or the like.

The exemplary embodiments of the disclosure have been described in detail, but the scope of the disclosure is not limited thereto and various variants and modifications by a person skilled in the art using a basic concept of the disclosure defined in claims also belong to the scope of the disclosure.

**Claims**

1. An image analysis device comprising:

   a memory; and
   a processor configured to execute instructions stored in the memory,
   wherein the processor is configured to:

   detect an indeterminate region or an abnormal region from an input medical image using an artificial intelligence (AI) model trained to detect a suspicious region and a lesion region in medical images; and
   determine the input medical image as a normal case when the indeterminate region or the abnormal region is not detected in the input medical image.

2. The image analysis device of claim 1, wherein the AI model is trained to detect the suspicious region using suspicious images and non-suspicious images and to detect the lesion region using lesion images including a target lesion and non-lesion images not including the target lesion, and

wherein the suspicious images include the lesion images and images including a lesion suspected as the target lesion.

3. The image analysis device of claim 1, wherein the processor is configured to:

obtain a suspiciousness score related to a confidence level at which the suspicious region is certain and a lesion score related to a confidence level at which the lesion region is certain, using the AI model; and
classify regions of the input medical image into one of normal, indeterminate, or abnormal based on analysis results of the input medical image including the suspiciousness score and the lesion score.

4. The image analysis device of claim 3, wherein the processor is configured to
determine the input medical image as the normal case when the entire regions of the input medical image are classified as being normal.

5. The image analysis device of claim 3, wherein the processor is configured to:

calculate a rearrangement score based on the suspiciousness score and the lesion score; and
obtain a classification result corresponding to the rearrangement score by using a first threshold value that classifies each region as being normal or indeterminate and a second threshold value that classifies each region as being indeterminate or abnormal.

6. The image analysis device of claim 5, wherein the processor is configured to
determine a highest rearrangement score analyzed from the input medical image as an abnormality score of the corresponding medical image.

7. The image analysis device of claim 1, wherein the processor is configured to
provide analysis results and normal case information for the input medical image obtained by the AI model to a designated device.

8. The image analysis device of claim 7, wherein the processor is configured to

provide the analysis results for the input medical image in at least one of forms of secondary capture (SC) of a DICOM format or grayscale softcopy presentation state (GSPS), and
wherein the SC or the GSPS for the normal case includes a graphical indicator indicating that the input medical image is the normal case, or
the SC or the GSPS for an abnormal case displays the indeterminate region and the abnormal region distinguishably.

9. A method of operating an image analysis device, the method comprising:

classifying regions of an input medical image into one of normal, indeterminate, or abnormal region using an artificial intelligence (AI) model trained to detect a suspicious region and a lesion region in a medical image, and determining the input medical image as a normal case when the indeterminate region or the abnormal region is not detected in the input medical image.

10. The method of claim 9, wherein the AI model is trained to detect the suspicious region using suspicious images and non-suspicious images and to detect the lesion region using lesion images including a target lesion and non-lesion images not including the target lesion, and
wherein the suspicious images include the lesion images and images including a lesion suspected of being the target lesion.

11. The method of claim 9, wherein the classifying regions of the input medical image comprises:

obtaining a suspiciousness score related to a confidence level at which the suspicious region is certain and a lesion score related to a confidence level at which the lesion region is certain, using the AI model; and
classifying regions of the input medical image into one of normal, indeterminate, or abnormal based on analysis results of the input medical image including the suspiciousness score and the lesion score.

12. The method of claim 11, wherein the classifying regions of the input medical image comprises:

calculating a rearrangement score based on the suspiciousness score and the lesion score and obtaining a classification result corresponding to the rearrangement score by using a first threshold value that classifies each region as being normal or indeterminate and a second threshold value that classifies each region as being indeterminate or abnormal.

13. The method of claim 14, further comprising:
determining a highest rearrangement score analyzed from the input medical image as an abnormality score of the corresponding medical image.

14. The method of claim 9, further comprising:
providing analysis results and normal case information for the input medical image obtained by the AI model to a designated device.

15. The method of claim 14, wherein the analysis results for the input medical image are provided in at least one of forms of secondary capture (SC) of a DICOM format or grayscale softcopy presentation state (GSPS), and wherein the SC or the GSPS for the normal case includes a graphical indicator indicating that the input medical image is the normal case.

# FIG. 1

10

Medical images

300

Image analysis device

| AI model 1 | AI model 2 |

310    320

Image storage device
(e.g., PACS)

200

100

User terminal

Viewer

110

# FIG. 2

310

Medical image

AI model 1

Suspiciousness score

330

Analysis result integrator

Analysis result: rearrangement score,
normal case information,
indeterminate region information,
abnormal (lesion) region information,
abnormality score, medical indicators, etc.

AI model 2

lesion(e.g., cancer) score

320

Normal | Indeterminate | Abnormal

Rearrangement score

0    First threshold value    Second threshold value    100

EP 4 564 364 A1

# FIG. 3

400A

410A — Medical image

Abnormality Score ⓇN ⓁN Composition Category **B** BI-RADS Density

430A  420A

EP 4 564 364 A1

# FIG. 4

400B

410B

Medical image

N

440B

Abnormality Score ⓡN ⓛN Composition Category **B** BI-RADS Density

430B    420B

# FIG. 5

EP 4 564 364 A1

# FIG. 6

450-1

450-2

450-3

Single Colormap

Contour map

Combined map

# FIG. 7

500

- Select the AI analyzed DICOM format

☑ SC Secondary Capture

Display Mode

⊙ Color Map ○ Singer Color Map ○ Grayscale Map ○ Combined Map

Result Type
INSIGHT Map

FIG. 8

<u>600</u>                                        <u>610</u>        <u>620</u>

| No. | Name | Patient ID | Age | Modality | Image | Data | Time | Manufacturer | Abnormaltiy Score | No Visible Abnormaltiy |
|---|---|---|---|---|---|---|---|---|---|---|
| 1 | Anonymized | 1XXXXXXXX | 26 | DR | 4 | 2023-04-24 | 10:00:00 AM | Hologic(1), Lunit(1) | N |  |
| 2 | Anonymized | 2XXXXXXXX | 33 | DR | 4 | 2023-04-24 | 11:00:00 AM | Hologic(1), Lunit(1) | 75 |  |
| 3 | Anonymized | 3XXXXXXXX | 35 | DR | 4 | 2023-04-24 | 12:00:00 AM | GE(1), Lunit(1) | LOW |  |
| 4 | Anonymized | 4XXXXXXXX | 45 | DR | 4 | 2023-04-24 | 09:00:00 AM | GE(1), Lunit(1) | 99 |  |
| 5 | Anonymized | 5XXXXXXXX | 69 | DR | 4 | 2023-04-24 | 10:00:00 AM | Simense(1), Lunit(1) | N |  |
| 6 | Anonymized | 6XXXXXXXX | 44 | DR | 4 | 2023-04-24 | 11:00:00 AM | Simense(1), Lunit(1) | 25 |  |

# FIG. 9

700

## Pre-populated Report

710

MAMMOGRAPHY

720

[History] For screening

[Density category] A
The breasts are almost entirely fatty.
There are no remarkable mass/suspicious microcalcifications.
There is no axillary lymphadenopathy.

[Impression] Negative Finding

[BI-RADS category] 1

[Recommendation] Routine mammography screening.
If you find a lump or other change, talk to your health care provider about it as soon as possible.

Cencel          Approve

730

# FIG. 10

800

Configuration setting

☑ SWIFT SCAN
  ☑ Indeterminate
  ☑ Report Auto-generation

Auto generated report setting

810 {
Report Title                                          (11/80)

Mammography

}

820 {
| DensityA | DensityB | DensityC | DensityD |

Description                                           (396/2000)

[History] For screening

[Density category] A
The breasts are almost entirely fatty.
There are no remarkable mass/suspicious microcalcifications.
There is no axillary lymphadenopathy.

[Impression] Negative Finding

[BI-RADS category] 1

[Recommendation] Routine mammography screening.
If you find a lump or other change, talk to your health care provider
about it as soon as possible.

}

# FIG. 11

900

EP 4 564 364 A1

**MMG** Worklist

| N | – – | Modality | Abnormality score | Breast density | comments |
|---|---|---|---|---|---|
| 1 | | MMG | 99 | B | |
| 2 | | MMG | 99 | B | |
| 3 | | MMG | 0 | B | No visible abnormality |
| 4 | | MMG | 0 | B | No visible abnormality |

910

Worklist
CXR
DBT
**MMG**

Patient History
_____
_____

| | | | | |
|---|---|---|---|---|
| N | Image | Image | Image | Image |

921   920

Report

[History] ------
[Density category] B
--------
--------
[Impression] Negative finding
[BI-BADS category] 1
[Recommendation]---
-----   --------

930

1000    FIG. 12

# FIG. 13

Obtain analysis results including suspiciousness score and lesion score for medical image by using AI model trained to detect suspicious region and lesion region in the medical image ⟩～S110

Classify regions of medical image into one of normal, indeterminate, or abnormal based on analysis results including suspiciousness score and the lesion score for medical image ⟩～S120

Determine medical image as normal case when entire regions of medical image are normal and determine medical image as abnormal case when medical image include either indeterminate region or abnormal region ～S130

Provide analysis results for medical image obtained by AI model and final analysis result including normal case information to designated device ⟩～S140

FIG. 14

| | |
|---|---|
| Store phrases set by user in report setting interface | ～S210 |

↓

| | |
|---|---|
| Obtain analysis result of medical image classified as normal case and generate report on medical image using phrases set in advance according to analysis result | ～S220 |

FIG. 15

| Display worklist for image reading task in conjunction with image storage device that store analysis results for medical images | ～S310 |

| In response to selection of first medical image, which is normal case, from worklist, display first medical image with graphical indicator indicating normal case | ～S320 |

| In response to selection of second medical image, which is abnormal case, from worklist, display second medical image with at least one of indeterminate region or lesion region | ～S330 |

Europäisches
Patentamt

European
Patent Office

Office européen
des brevets

## EUROPEAN SEARCH REPORT

**Application Number**

EP 24 20 3402

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/180514 A1 (VLASIMSKY RICHARD [US]) 9 June 2022 (2022-06-09) * paragraph [0053] - paragraph [0076]; figures 1-4b * | 1-15 | INV. G16H30/40 G16H50/20 |
| X | WO 2022/178176 A1 (KOIOS MEDICAL INC [US]; BARINOV LEV [US]; JAIRAJ AJIT [US]) 25 August 2022 (2022-08-25) * paragraph [0070] - paragraph [0089]; figures 4-6 * * paragraph [0111] - paragraph [0120]; figures 8, 9A, 9B, 10A, 10B * | 1-15 | |
| X | US 2020/315589 A1 (STAVROS ANTHONY THOMAS [US] ET AL) 8 October 2020 (2020-10-08) * paragraph [0212] - paragraph [0243]; figure 4A * * paragraph [0462] - paragraph [0470]; figure 11B * | 1-15 | |
| X | WO 2022/221712 A1 (CUREMETRIX INC [US]) 20 October 2022 (2022-10-20) * paragraph [0263] * * paragraph [0486] * * paragraph [0505] - paragraph [0507]; figures 33-35 * | 1-15 | **TECHNICAL FIELDS SEARCHED (IPC)** G16H |

The present search report has been drawn up for all claims

| Place of search | Date of completion of the search | Examiner |
|---|---|---|
| Munich | 6 March 2025 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 3402

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2025

| Patent document cited in search report | Publication date | Patent family member(s) | | Publication date |
|---|---|---|---|---|
| US 2022180514 A1 | 09-06-2022 | AU | 2021396186 A1 | 27-07-2023 |
| | | CA | 3204907 A1 | 16-06-2022 |
| | | EP | 4260236 A1 | 18-10-2023 |
| | | US | 2022180514 A1 | 09-06-2022 |
| | | US | 2024346654 A1 | 17-10-2024 |
| | | US | 2024346655 A1 | 17-10-2024 |
| | | WO | 2022125640 A1 | 16-06-2022 |
| WO 2022178176 A1 | 25-08-2022 | AU | 2022223708 A1 | 07-09-2023 |
| | | CA | 3208656 A1 | 25-08-2022 |
| | | EP | 4294261 A1 | 27-12-2023 |
| | | IL | 305254 A | 01-10-2023 |
| | | JP | 2024507820 A | 21-02-2024 |
| | | US | 2024013384 A1 | 11-01-2024 |
| | | WO | 2022178176 A1 | 25-08-2022 |
| US 2020315589 A1 | 08-10-2020 | NONE | | |
| WO 2022221712 A1 | 20-10-2022 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230168898

- KR 1020240068620